# EUROPEAN PATENT APPLICATION

(11) **EP 2 402 355 A1**
(43) Date of publication of application: **04.01.2012**
(21) Application number: 10168153.4
(22) Date of filing: 01.07.2010
(51) Int. Cl.: C07H 17/00, A61K 31/7052, A61P 31/04

(54) **Crystalline anhydrous forms II and III of Tulathromycin**

(71) Applicant: Novartis AG, 4056 Basel (CH)
(72) Inventor: Hotter, Andreas, 6250 Kundl, Austria (AT); Pichler, Arthur, 6250 Kundl, Austria (AT); Langes, Christoph, 6020 Innsbruck, Austria (AT); Ludescher, Johannes, 6250 Kundl, Austria (AT); Garcia, Rafael, 08400 Granollers, Spain (ES); Martorell, Oriol, 08400 Granollers, Spain (ES); Codony, Albert, 08400 Granollers, Spain (ES)
(74) Representative: Liphardt, Bernd

(57) **Abstract**

The invention relates to crystalline forms (II and III) of Tulathromycin of formula (I) and to methods for the preparation of the same. The present inventon, in addition, relates to the use of crystalline forms (II and III) of Tulathromycin as an anti-infective medicament for treating bacterial infections in animals.

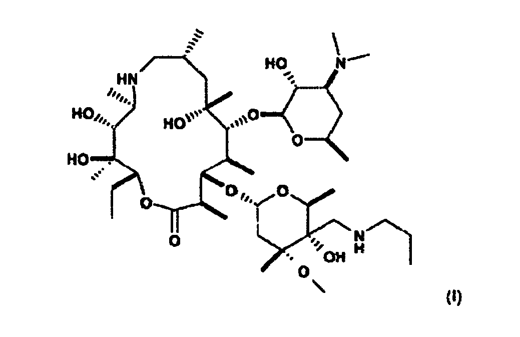

## Description

The present invention relates to novel crystalline forms of Tulathromycin and to methods for the preparation of the same. Furthermore the present invention relates to the use of the novel crystalline forms as intermediates for the formulation of an anti-infective medicament. Moreover the present invention relates to the use of the novel forms for the preparation of an anti-infective medicament. Finally the present invention relates to pharmaceutical compositions comprising the novel crystalline forms in an effective amount and to the use of the novel crystalline forms of Tulathromycin as an anti-infective medicament.

### BACKGROUND OF THE INVENTION

Tulathromycin, is a semi-synthetic macrolid antibiotic. Due to the presence of three amine groups in its chemical structure Tulathromycin belongs to the triamilide subclass distinguishing its structure from azalides, ketolides and other macrolides. It is existent as a mixture of two isomers:
a) Tulathromycin A with the chemical name (2*R*,3*S*,4R,5*R*,8*R*,10*R*,11*R*,12*S*,13*S*,14*R*)-13-[[2,6-Dideoxy-3-*C*-methyl-3-*O*-methyl-4-*C*-[(propylamino)methyl]-α-L*-ribo-*hexopyranosyl]oxy]-2-ethyl-3,4,10-trihydroxy-3,5,8,10,12,14-hexamethyl-11-[[3,4,6-trideoxy-3-(dimethylamino)-β-D-xylo-hexopyanosyl]oxy]-1-oxa-6-azacyclopentadecan-15-one (Formula I); 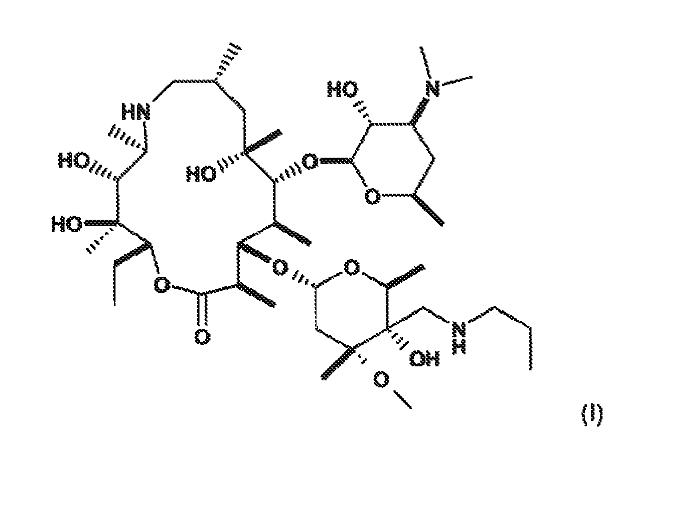
b) Tulathromycin B with the chemical name (2*R*,3*R*,6*R*,8*R*,9*R*,10*S*,11*S*,12*R*)-11-[[2,6-Dideoxy-3-*C*-methyl-3-*O*-methyl-4-*C*-[(propylamino)methyl]-α-L-ribo-hexopyranosyl]oxy]-2-[(1*R*,2*R*)-1,2-dihydroxy-1-methylbutyl]-8-hydroxy-3,6,8,10,12-pentamethyl-9-[[3,4,6-trideoxy-3-(dimethylamino)-β-D-*xylo*-hexopyanosyl]oxy]-1-oxa-4-azacyclotridecan-13-one (Formula II);

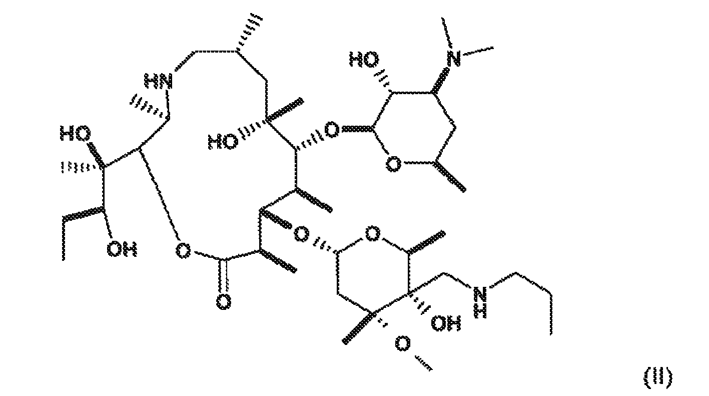

In solution, the two structural isomers form a stable equilibrated mixture with a ratio of about 90:10 (%Tulathromycin A : %Tulathromycin B).

Tulathromycin blocks bacterial protein biosynthesis by selectively binding to bacterial 50S ribosomal subunits it is marketed by Pfizer under the brand name Draxxin^{®} Draxxin^{®} solution for injection is currently indicated for the treatment of bacterial respiratory diseases in cattle and pigs. It is used exclusively for veterinary medicine.

WO 98/56802 discloses the compound Tulathromycin per se or a pharmaceutically acceptable salt thereof. The specification discloses the preparation of suitable salts but is silent about any crystalline form of Tulathromycin free base.

WO 01/02414 describes crystalline and liquid crystalline polymorphs of Tulathromycin diphosphate, a process for their manufacture and a process for purifying Tulathromycin via the diphosphate polymorphs.

WO 00/69874 discloses crystalline forms ot Tulathromycin free base such as an anhydrous form, the monohydrate and the sesquihydrate.

Polymorphism is a phenomenon relating to the occurrence of different crystal forms for one molecule. There may be several different crystalline forms for the same molecule with distinct crystal structures and varying physical properties like melting point, XRPD-spectrum and IR-spectrum, These polymorphs are thus distinct solid forms which share the molecular formula of the compound from which the crystals are made up. However, they may have distinct advantageous physical properties like stability, dissolution, bioavailability, hygroscopicity and/ or purity.

It has turned out that Tulathromycin monohydrate looses its water below 100°C, and Tulathromycin sesquihydrate readily looses water at ambient conditions and is polimorphically extremely unstable, as described in WO 00/69874. In addition, Tulathromycin anhydrate according to WO 00/69874 is assumed to contain some residual solvent as EMEA's scientific discussion CVMP/0968/03 (page 6) mentions a 0.5 % limit for heptane in the active pharmaceutical ingredient. The presence of solvents is, however, highly undesirable. Accordingly, there is a need for novel crystalline forms of Tulathromycin which overcome the above-mentioned drawbacks.

Surprisingly, novel crystalline forms of Tulathromycin were found which show different unpredictable properties compared to the crystalline forms enclosed in WO 00/69874. E.g the novel forms show different XRPD- and IR-spectra as well as different DSC- TGA- and moisture sorption/ desorption-curves.

In addition, the novel crystalline form II of Tulathromycin of the present invention is thermally more stable than Tulathromycin monohydrate and Tulathromycin sesquihydrate described in WO 00/69874. Furthermore the novel crystalline form II is also polymorphically more stable within a broad range of relative humidity compared to the monohydrate and the sesquihydrate.

Moreover, the novel form II of the present invention is free from any residual solvents as residual solvents are easily removable by practical manufacturing techniques due to the unique properties of the present novel form.

The novel form II of Tulathromycin of the present invention easily takes up limited amounts of water at increased relative humidity. The water is also quickly lost again when subjecting the novel polymorph II to decreased relative humidity, Tulathromycin form II is chemically and polymorphically stable independent of its water content, e.g. it does not change its crystal structure at ambient conditions for prolonged time periods.

Form II of Tulathromycin represents an anhydrous crystalline form of Tulathromycin in high purity, free from any residual solvents, containing, for example, from about 0 % to 2.0 % of water.

Polymorph II of the present invention may be used as starting material for pharmaceutical formulations as well as active ingredient in pharmaceutical preparations.

II another embodiment of the invention a novel polymorph III of Tulathromycin is provided. The novel polymorph III of the present invention is an anhydrous form of Tulathromycin containing up to 0 5 % of water and up to 0.5 % of residual solvents. Form III of Tulathromycin is the intermediate in the manufacture of polymorph II of Tulathromycin, but may be used as starting material for pharmaceutical formulations or as active ingredient in pharmaceutical preparations as well.

### SUMMARY OF THE INVENTION

In one embodiment, the present invention refers to a novel crystalline form of Tulathromycin, The novel crystalline form is anhydrous and designated as anhydrous form II.

Anhydrous form II of Tulathromycin can be characterized by an XRPD pattern comprising peaks at 2-theta angles of 5.3 ± 0.2°, 6.7 ± 0,2°, 8.2 ± 0.2°, 8.8 ± 0,2°, 9.5 ± 0,2°, 10.9 ± 0.2°, 11.1 ± 0.2°, 13.0 ± 0.2°, 13.2 ± 0.2°, 14.8 ± 0,2°, 15.2 ± 0.2°, 16.5 ± 0.2°, 18.9 ± 0.2°, 19.4 ± 0.2° and 20.2±0.2°.

Alternatively anhydrous form II of Tulathromycin can be described by an IR spectrum comprising peaks at wavenumbers of 2934 ± 2 cm⁻¹, 1734 ± 2 cm⁻¹, 1456 ± 2 cm⁻¹, 1376±2 cm⁻¹, 1331 ± 2 cm⁻¹, 1270 ± 2 cm⁻¹, 1174 ± 2 cm⁻¹, 1106 ± 2 cm⁻¹, 1053 ± 2 cm⁻¹, 996 ± 2 cm^{- 1}, 972 ± 2 cm⁻¹, 898 ± 2 cm⁻¹, 858 ± 2 cm⁻¹, 834 ± 2 cm⁻¹, 805 ± 2 cm⁻¹, 697 ± 2 cm⁻¹ and 647 ± 2 cm⁻¹,

A further embodiment of the invention concerns a process for the preparation of anhydrous form II of Tulathromycin comprising the step of subjecting form III of Tulathromycin to a relative humidity (RH) of about 40 % - 100 %.

Another embodiment of the present invention concerns a process for the preparation of anhydrous form II of Tulathromycin comprising the steps of:
a) mixing Tulathromycin with a dry solvent at ≤ 10 °C;
b) isolating the solid material;
c) drying the isolated material to obtain form III of Tulathromycin;
d) removing residual solvents by subjecting the material to an atmosphere having about 40-100 % RH; and
e) optionally removing water by subjecting the material to an atmosphere having 0-40 % RH;

Moreover, the present invention relates to the use of anhydrous form II of Tulathromycin as an intermediate for the formulation of an anti-infective medicament.

In addition, the present invention relates to the use of anhydrous form II of Tulathromycin for the preparation of an anti-infective medicament.

Another aspect of the present invention relates to a pharmaceutical composition comprising an effective amount of the anhydrous form II of Tulathromycin and optionally a pharmaceutically acceptable carrier.

The pharmaceutical composition can be used for treating bacterial infections in animals. In particular, it can be employed for treating or preventing bacterial respiratory diseases in cattle and pigs.

In another embodiment the present invention relates to the use of anhydrous form II as an anti-infective medicament.

According to a further embodiment, the present invention refers to another novel crystalline form of Tulathromycin The novel crystalline form is anhydrous and designated as anhydrous form III.

Anhydrous form III of Tulathromycin can be characterized by an XRPD pattern comprising peaks at 2-theta angles of 6.0 ± 0.2°, 6.2 ± 0.2°, 6.4 ± 0.2°, 6.7 ± 0.2°, 7.6 ± 0.2°. 8.1 ± 0.2°, 8.2 ± 0.2°, 8.4 ± 0.2°, 8.9 ± 0.2°, 9.2 ± 0.2°, 9.3 ± 0.2°, 9.5 ± 0.2°, 10.9 ± 0.2°. 11.1 ± 0.2°, 13.0 ± 0.2°, 13.2 ± 0.2°, 14.2 ± 0.2°, 15.1 ± 0.2°. 15.4 ± 0.2°, 16.1 ± 0.2°, 16.4 ± 0.2°, 16.9 ± 0,2°, 18.9 ± 0.2°, 19.1 ± 0.2°, 19.4 ± 0,2° and 20.1 ± 0.2°.

The present invention according to another embodiment concerns a process for the preparation of anhydrous form III of Tulathromycin comprising the steps of:
a) mixing Tulathromycin with a dry solvent at ≤ 10 °C;
b) isolating the solid material; and
c) drying the isolated material to obtain form III of Tulathromycin.

In another aspect the invention relates to the use of Tulathromycin form III in the manufacture of Tulathromycin form II.

Moreover the present invention relates to the use of anhydrous form III of Tulathromycin as an intermediate for the formulation of an anti-infective medicament.

In addition the present invention relates to the use of anhydrous form III of Tulathromycin for the preparation of an anti-infective medicament.

Tulathromycin form III is polymorphically stable up to a relative humidity of about 50 %. preferably up to a relative humidity of about 40 %. Above about 40 % relative humidity form III of Tulathromycin converts to form II of Tulathromycin.

Other objects, features. advantages and aspects of the present invention will become apparent to those of skill from the following description. It should be understood, however, that the description and the following specific examples, while indicating preferred embodiments of the invention, are given by way of illustration only. Various changes and modifications within the spirit and scope of the disclosed invention will become readily apparent to those skilled in the art from reading the description and from reading the other parts of the present disclosure.

### BRIEF DESCRIPTION OF THE DRAWINGS

Figure 1: XRPD pattern of anhydrous form II of Tulathromycin
Figure 2: IR spectrum of anhydrous form II of Tulathromycin
Figure 3: DSC curve of anhydrous form II of Tulathromycin
Figure 4: TGA curve of anhydrous form II of Tulathromycin
Figure 5: Dynamic moisture sorption/desorption curve of anhydrous form II of Tulathromycin
Figure 6: TGA curve of anhydrous form III of Tulathromycin
Figure 7: XRPD pattern of anhydrous form III of Tulathromycin
Figure 8: Dynamic moisture sorption/desorption curve of anhydrous form III of Tulathromycin
Figure 9: Dynamic moisture sorption/desorption curve of Tulathromycin monohydrate according to WO 00/69874

### DETAILED DESCRIPTION OF THE INVENTION

The term "room temperature" as used herein indicates that the applied temperature is not critical and that no exact temperature value has to be kept. Usually, "room temperature" is understood to mean temperatures of about 15 °C to about 25 °C [see European Pharmacopoeia 6.6, 1.2 (2010)].

The term "dry solvent" used herein means that the solvent is water free, containing less than about 0.1 % water, preferably less than about 0.05 % when measured by Karl Fisher.

The term "wet atmosphere" as used herein refers to an atmosphere having a relative humidity (RH) above about 50 %.

The term "dry atmosphere" as used herein refers to an atmosphere having a relative humidity below about 40 %.

The present invention relates to the novel crystalline anhydrous form II of Tulathromycin as well as to a process for its manufacture comprising the step of subjecting form III of Tulathromycin to a relative humidity of about 40-100 %.

As mentioned before a further embodiment of the invention concerns a process for the preparation of anhydrous form II of Tulathromycin comprising the steps of:
a) mixing Tulathromycin with a dry solvent at ≤ 10 °C;
b) isolating the solid material;
c) drying the isolated material to obtain form III of Tulathromycin;
d) removing residual solvents by subjecting the material to an atmosphere having 40-100 % RH; and
e) optionally removing water by subjecting the material to an atmosphere having 0-40 % RH;

Any non-hydrated form of Tulathromycin may be applied in step a) of the above process. Suitable forms are e g. amorphous Tulathromycin, crystalline anhydrous Tulathromycin or mixtures thereof. A suitable crystalline anhydrous form is e.g. Tulathromycin anhydrate as disclosed in WO 00/69874 If amorphous Tulathromycin is used as starting material, surface adsorbed water is preferably removed by any conventional method such as drying at elevated temperatures or storing the material at dry atmospheres (e.g. over P₂O₅).

In the crystallization step of Tulathromycin form III, Tulathromycin is preferably used at a concentration ranging from 200 to 800 g/l, more preferably from 400 to 600 g/l and most preferably the concentration is 500 g/l,

The Tulathromycin starting material is in admixture with a solvent or solvent mixture. Any type of solvent can be employed as long as it does not adversely effect the formation of the desired crystalline anhydrous form III. A suitable solvent for the crystallization step is isobutylmethyl ketone. However, it is crucial that the water content of the solvent or solvent mixture used is carefully controlled, Normally a water content of less than about 0.5%, preferably a water content of less than about 0.1 %, each determined by Karl Fischer titration in the solvent or solvent mixture is used in the crystallization step.

The mixture comprising the Tulathromycin starting material and the solvent or mixture of solvents is stirred until all of the applied material has converted to the desired anhydrous form III of Tulathromycin; in general, a stirring time of, for example, about 1 to 48 hours, more preferably of about 1 to 24 hours and most preferably of about 1 to 20 hours is sufficient. Preferably, the temperature does not exceed about 10 °C during the conversion.

The temperature in the crystallization step is advantageously kept at or below about 10 °C,

After complete transformation the solid is isolated from the reaction mixture by e.g. filtration or centrifugation.

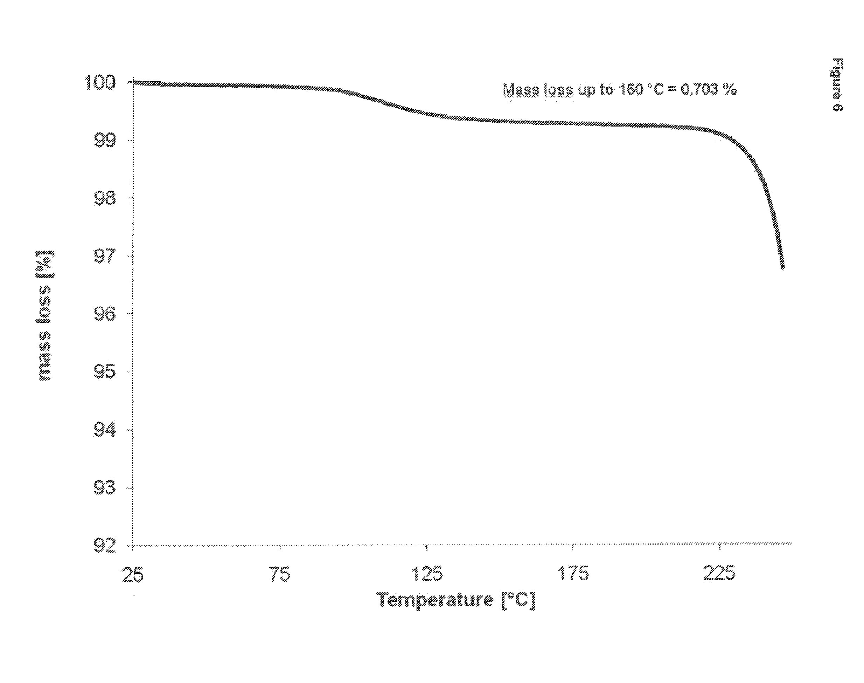

The isolated material is then dried. Preferably drying is performed under vacuum and at a temperature preferably ranging from about 20°C to 60 °C, more preferably from about 20°C to 40 °C and most preferably drying is performed at room temperature. Form III of Tulathromycin contains typically less than about 0.5 % of water, e.g. about 0.3 to 0.4 %, determined by KF and less than about 0.5 % of organic solvents, e.g. about 0.3 to 0.4 % of isobutylmethyl ketone when isobutylmethyl ketone is used as solvent, e.g. as demonstrated by the TGA curve of figure 6 a mass loss of about 0.7 % up to 160 °C is observed The mass loss of 0.7 % consists of 0.4 % water and 0.3 % isobutylmethyl ketone.

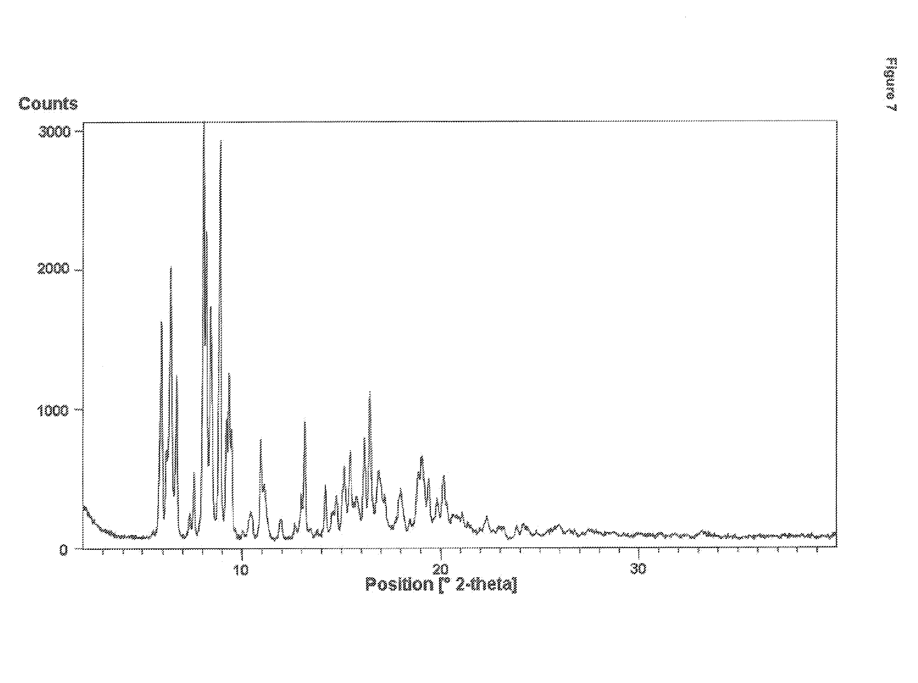

Tulathromycin form III of the present invention is polymorphic stable when stored at about less than 40 % relative humidity. Furthermore Tulathromycin polymorph III is chemically stable. The thus pure crystalline anhydrous form III of Tulathromycin can be characterized by an X-ray powder diffraction (XRPD) pattern having peaks at 2-theta angles of 6.0 ± 0.2°, 6.2 ± 0 2°, 6.4 ± 0.2°, 6.7 ± 0.2°, 7.8 ± 0.2°, 8,1 ± 0.2°, 8.2 ± 0.2°, 8.4 ± 0.2°. 8.9 ± 0.2°, 9.2 ± 0.2°, 9.3 ± 0.2°, 9.5 ± 0.2°, 10.9 ± 0.2°, 11.1 ± 0.2°, 13.0 ± 0.2°, 13.2 ± 0.2°, 14,2 ± 0.2°, 15.1 ± 0.2°, 15.4 ± 0.2°, 16.1 ± 0.2°, 16.4 ± 0.2°, 16.9 ± 0.2°, 18.9 ± 0.2°, 19.1 ± 0.2°, 19.4 ± 0.2° and 20.1 ± 0.2°. A characteristic XRPD pattern is displayed in figure 7.

The preparation of form II of Tulathromycin comprises the exposure of Tulathromycin form III to a wet humidity. The wet humidity is defined as a RH of preferably 40-100 %, more preferably 60-95 % and most preferably 70-90 %. The exposure to the wet humidity results in an exchange of isobutylmethyl ketone against water and the conversion of form III of Tulathromycin to form II of Tulathromycin. The material is preferably subjected to the wet atmosphere for about 1-168 hours, more preferably for about 1-72 hours and most preferably for about 1-24 hours.

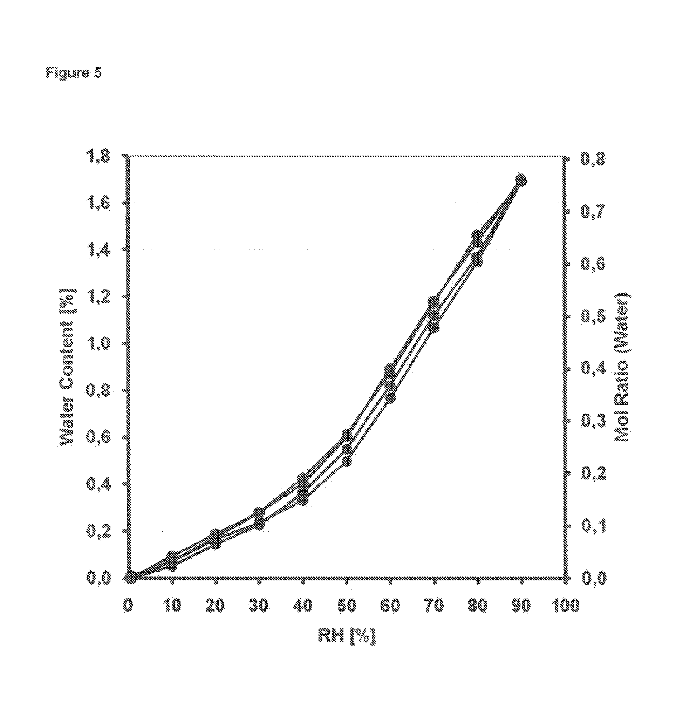

Tulathromycin form II represents an anhydrous form, but may nevertheless contain up to 2 0 % of water, e.g. 0-1.7 %. The water content of Tulathromycin form II may be decreased by subjecting form II to a dry atmosphere with a RH of preferably 0-40 %, more preferably 0-30 % and most preferably 0-20 %. The material is preferably subjected to the dry atmosphere for about 1-72 hours, more preferably for about 1-48 hours and most preferably for about 1-24 hours (see also moisture sorption curve figure 5).

Tulathromycin form II is also chemically stable independent of its water content. It is also polymorphically stable independent of its water content, e.g. it does not change its crystal structure at ambient conditions even after prolonged time periods.

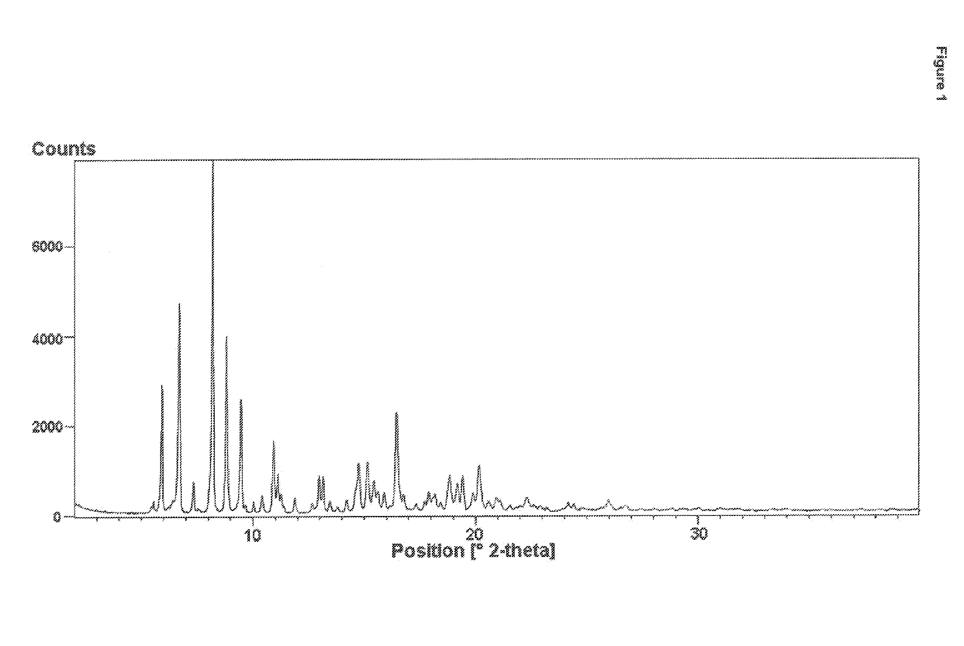

The thus obtained pure crystalline anhydrous form II of Tulathromymn can be characterized by an X-ray powder diffraction (XRPD) pattern having peaks at 2-theta angles of 5.9 ± 0.2°, 6.7 ± 0.2°, 8.2 ± 0.2°, 8.8 ± 0.2°, 9 5 ± 0.2°, 10 9 ± 0.2°, 11.1 ± 0.2°, 13.0 ± 0.2°, 13.2 ± 0.2°, 14.8 ± 0.2°, 15.2 ± 0.2°, 16.5 ± 0.2°, 18.9 ± 0.2°, 19.4 ± 0.2° and 20.2 ± 0.2°. A charactenstic XRPD pattern is displayed in Figure 1.

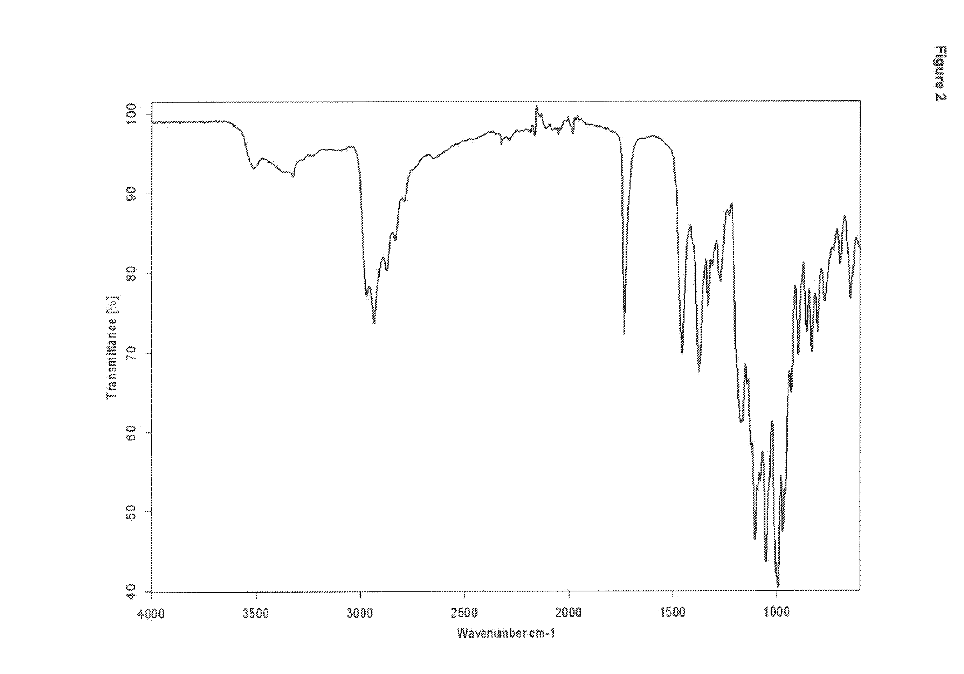

In addition, anhydrous form II of Tulathromycin can be characterized by an infrared (IR) spectrum having characteristic bands at 2934 ± 2 cm⁻¹, 1734 ± 2 cm⁻¹, 1456 ± 2 cm⁻¹, 1376 ± 2 cm⁻¹, 1331 ± 2 cm⁻¹, 1270 ± 2 cm⁻¹, 1174 ± *2 cm⁻¹* 1106 ± 2 cm⁻¹, 1053 ± 2 cm⁻¹, 996 ± 2 cm⁻¹, 972 ± 2 cm⁻¹, 898 ± 2 cm⁻¹, 858 ± 2 cm⁻¹, 834 ± 2 cm⁻¹, 805 ± 2 cm⁻¹, 697 ± 2 cm⁻¹ and 647 ± 2 cm⁻¹, A typical IR spectrum is shown in Figure 2.

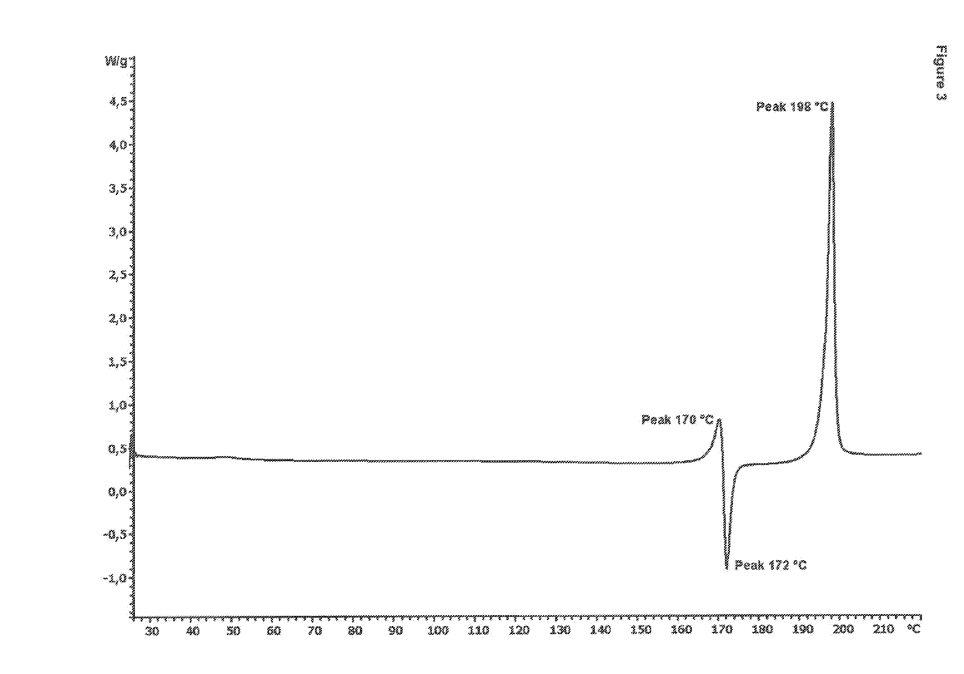

Moreover anhydrous form II of Tulathromycin can be specified by the differential scanning calorimetric (DSC) curve shown in figure 3. Anhydrous form II of Tulathromycin shows an endotherm with a peak maximum at about 170 °C, which is due to the melting process. Immediately after the 1^{st} endotherm an exotherm with a peak maximum at about 172 °C due to the crystallization of Tulathromycin anhydrate as described in WO 00/69874 follows. A 2^{nd} endotherm with a peak maximum at about 198 °C is due to the melting process of Tulathromycin anhydrate of WO 00/69874.

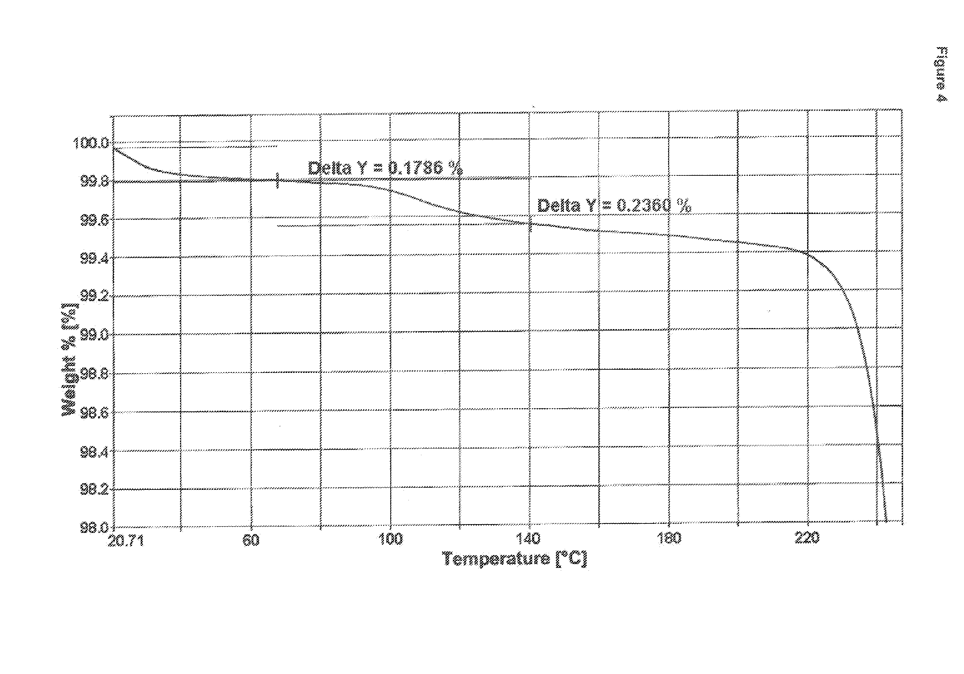

Figure 4 shows a characteristic thermogravimetric analysis (TGA) curve of pure crystalline anhydrous form II of Tulathromycin. The curve shows a total mass loss of about 0.4 w%, which appears in a two step process. In the first step, from the beginning of the measurement to about 65 °C. a decrease in mass of about 0.2 w% can be observed. Furthermore in the second step, from about 65 °C to about 140 °C, again a decrease in mass of about 0.2 w% appears. The total mass loss of 0.4 w% is due to the loss of water.

Finally the pure crystalline anhydrous form II of Tulathromycin can be characterized by the typical dynamic moisture sorption/ desorption curve displayed in Figure 5. As can be seen from Figure 5 crystalline anhydrous form II of Tulathromycin shows a reversible water uptake from about 0 % water at about 1 % RH to about 1.7 % of water at about 90 % RH,

The CVMP VICH guideline 18 "Impurities: residual solvents in new veterinary medicinal products, active substances and excipients" recommends acceptable amounts for residual solvents in veterinary pharmaceuticals. Since there is no therapeutic benefit from residual solvents, all residual solvents should be removed to the extent possible. In very many cases the solvents are not completely removed by practical manufacturing techniques. Thus there is a need for a crystalline form of Tulathromycin from which residual solvents are easily removable by practical manufacturing techniques, resulting in a crystalline form of Tulathromycin in high purity, free from any residual solvents.

Form III of Tulathromycin was analyzed by XRPD. It can be characterized by an XRPD pattern having peaks at 2-theta angles of 6.0 ± 0.2°,6.2 ± 0.2°, 6.4 ± 0.2°, 6,7 ± 0.2°, 7.6 ± 0.2°, 8 1 ± 0.2°, 8.2 ± 0.2°, 8.4 ± 0.2°, 8.9 ± 0,2°, 9.2 ± 0.2°, 9.3 ± 0,2°, 9.5 ± 0,2°, 10.9 ± 0.2°, 11.1 ± 0.2°, 13.0 ±0.2°, 13.2 ±0.2°,14.2±0.20°, 15.1 ±0.2°, 15.4±0.2°, 16.1 ±0.2°. 16.4 ± 0.2°, 16.9± 0,2°, 18.9 ± 0.2°.19.1 ± 0.2°,19.4 ± 0.2° and 20.1 ± 0.2°, A typical XRPD pattern of the anhydrous form III of Tulathromycin is shown in Figure 7.

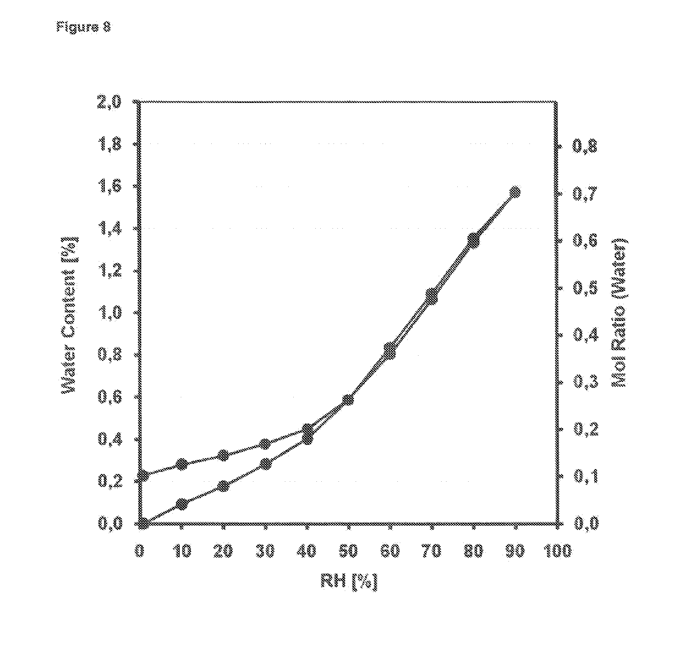

Anhydrous form III of Tulathromycin was subjected to a moisture sorption/ desorption experiment. The sorption cycle from about 1 % RH to about 90 % RH showed a water uptake from about 0.2 % to about 1.6 % which occurred in two steps: a slower water uptake in the first step from about 0-40 % RH and a faster water uptake in the second step from about 50-90 % RH. However, in the desorption cycle from about 90 % RH to about 1 % RH a continuous water loss from about 1.6 % to 0 % was observed. The difference between the sorption and the desorption curve from about 0-40 % RH is a proof for a change in the crystal structure. XRPD of the material after the sorption/ desorption experiment confirmed the structure change as the crystals were identified as pure crystalline anhydrous form II of Tulathromycin. The change in the crystal lattice is irreversible and due to an exchange of isobutylmethyl ketone against water, resulting in a residual solvent free crystalline form. A typical dynamic moisture sorption/ desorption curve of anhydrous form III of Tulathromycin is shown in Figure 8

Following the above described moisture sorption/ desorption experiment the TGA curve of the obtained material corresponds to the curve displayed in figure 4. The TGA curve shows a mass loss of 0.4 w% up to 140°C, which is due to the loss of water and proves the absence of residual solvent.

In EMEA's scientific discussion CVMP/0968/03 (page 6) the final purification step for Tulathromycin using heptane as solvent is mentioned, whereas Tulathromycin anhydrate is obtained. It is further stated that the specified limit of heptane at 0.5 % in Tulathromycin is considered qualified. Due to the given heptane limit it is assumed that there is still some residual solvent present.

Graph 7 of WO 00/69874 provides a typical moisture sorption/ desorption curve of the Tulathromycin anhydrate as described therein. It can be seen from graph 7 that Tulathromycin anhydrate according to WO 00/69874 practically shows no water uptake up to 90 % RH. It is this believed that an exchange of water against residual solvent, as described above for the anhydrous form III of the present invention, doesn't work with Tulathromycin anhydrate according to WO 00/69874.

WO 00/69874 disposes crystalline Tulathromycin monohydrate as well. According to WO 00/69874 the monohydrate starts to lose water and converts to a pseudopolymorphic form at about 70-75 °C. The DSC curve displayed in graph 4 of WO 00/69874 confirms this behavior. However, the anhydrous form II of the present invention shows no thermal event up to about 165 °C (see DSC curve in Figure 3 of the present invention) proving that anhydrous form II of Tulathromycin is thermally more stable than Tulathromycin monohydrate described in WO 00/69874.

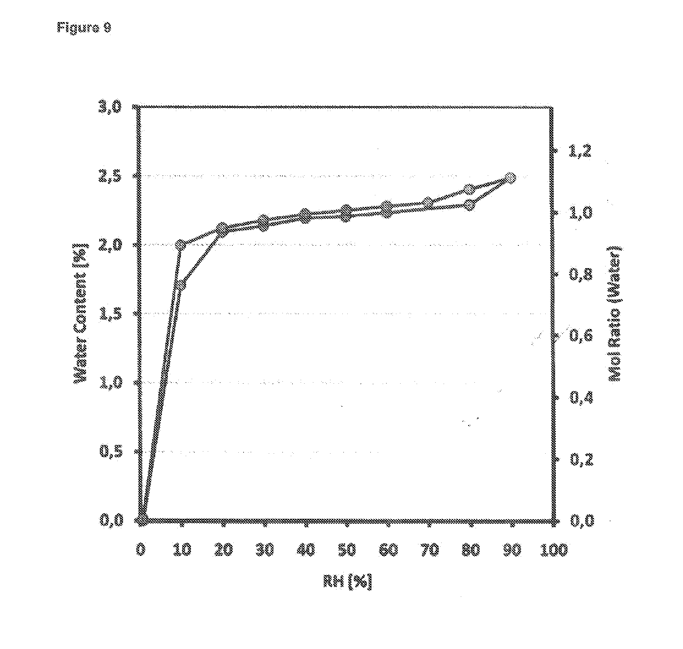

Furthermore a moisture sorption/ desorption experiment performed with Tulathromycin monohydrate according to WO 00/69874 shows a moisture sorption/ desorption curve as displayed in Figure 9. It can be seen from Figure 9 that Tulathromycin monohydrate according to WO 00/69874 loses its water between 0-10 % RH, proving that Tulathromycin monohydrate according to WO 00/69874 is not stable within 0-10 % RH. However, the moisture sorption/ desorption curve of anhydrous form II of Tulathromycin as displayed in Figure 5 does not show any phase transition but only slight changes in the water content. Thus crystalline anhydrous form II of Tulathromycin is stable within a range of 0-90 % RH, whereas Tulathromycin monohydrate according to WO 00/69874 loses its water when subjected to atmospheres having 0-10 % RH.

WO 00/69874 disposes crystalline Tulathromycin sesquihydrate as well. According to WO 00/69874 the sesquihydrate loses water under routine handling conditions (e.g. 25 °C/70 % RH). Furthermore WO 00/69874 mentions a loss of water at about 35 °C followed by the crystallization of Tulathmmycin anhydrate. Considering the above described thermal and moisture associated properties of the novel anhydrous form II, Tulathromycin sesquihydrate of WO 00/69874 is assumed to be the less stable form.

As a consequence crystalline anhydrous form II according to the present invention is deemed to be thermally more stable than the monohydrate and the sesquihydrate described in WO 00/69874. Furthermore the anhydrous form II of the present invention shows better stability within a range of 0-90 % RH compared to the monohydrate and the sesquihydrate.

In addition the novel crystalline anhydrous form 11 of Tulathromycin contains no residual solvent due to its unique behavior described in the moisture sorption/ desorption experiment herein, whereas the anhydrate of WO 00/69874 still contains some residual solvent according to EMEA's scientific discussion CVMP/0968/03 (page 6).

Further embodiments of the present invention relate to the use of anhydrous forms II and III of Tulathromycin (i) as intermediates for the fomulation of an anti-infective medicament, (ii) for the preparation of an anti-infective medicament, or (iii) as an anti-infective medicament.

Another aspect of the present invention relates to a pharmaceutical composition comprising effective amounts of the anhydrous forms II and III of Tulathromycin and optionally a pharmaceutically acceptable carrier.

Polymorphs and III of Tulathromycin may be formulated as crystalline forms II and III optionally in the presence of a crystalline or amorphous acid, preferably benzenesulfonic acid, D- or L-lactic acid, succinic acid, D- and L-tartaric acid, malic acid, fumaric acid, or benzoic acid, most preferably citric acid.

The isomer ratio of Tulathromycin A and B in the novel polymorphs II and III may vary depending on the amount of Tulathromycin A and B present in the starting material for the crystallization or recrystallization of form III respectively II. Typically, the crystals contain more than 90 % of isomer A and less than 10 % of Tulathromycin Isomer B; more typically crystalline forms II and III contain more than about 97 % Tulathromycin A and less than about 3 % of Tulathromycin B, in particular >98 % of Tulathromycin A and < 2 % of Tulathromycin B. The ratio Tulathromycin A: B may be determined by HPLC.

Pharmaceutical compositions comprising novel Tulathromycin polymorphs II and III can be used for treating bacterial infections in animals. In particular, they can be employed for treating or preventing bacterial respiratory diseases in cattle and pigs,

### EXAMPLES

XRPD patterns were obtained with an X'Pert PRO diffractometer (PANalytical, Almelo, The Netherlands) equipped with a thetaitheta coupled goniometer in transmission geometry, programmable XYZ stage with well plate holder, Cu-Ka_{1.2} radiation source (wavelength 0.15419 nm) with a focusing mirror, a 0.5° divergence slit, a 0.02° soller slit collimator and a 1° anti-scattering slit on the incident beam side, a 2 mm anti-scattering slit, a 0,02° soller slit collimator and a Nickel filter on the diffracted beam side and a solid state PIXrel detector. The patterns were recorded at a tube voltage of 40 kV, tube current of 40 mA, applying a stepsize of 0-013° 2-theta with 80 s per step in the angular range of 2° to 40" 2-theta,

The IR spectrum was collected on a MKII Golden Geta™ Single Rejection Diamond ATR (attenuated total reflection) cell with a Broker Tensor 27 FTIR spectrometer with 4 cm⁻¹ resolution at ambient conditions. To collect a spectrum a spatula tip of a sample was applied to the surface of the diamond in powder form. Then the sample was pressed onto the diamond with a sapphire anvil and the spectrum was recorded. A spectrum of the clean diamond was used as background spectrum. A typical precision of the wavenumber values is in the range of about ± 2 cm ⁻¹, Thus, an infrared peak that appears at 1716 cm⁻¹ can appear between 1714 and 1718 cm⁻¹ on most infrared spectrometers under standard conditions.

TGA's were performed with Thermogravimetric-system TGA-7, Pyris-Software for Windows NT, (Perkin-Elmer, Norwalk, Cl., USA), Platinum-sample holder (50 µl), Nitrogen as the purge gas (Sample purge: 20 ml/min, balance purge. 40 ml/min). Heating ate: 10 ^{°}C/min;

The moisture sorption/ desorption isotherms were recorded with a SPS-11 moisture sorption analyzer (MD Messtechnik, Ulm, D). In the experiment displayed in figure 5 the measurement cycle was started at 0 % RH increased in 10 % steps up to 90 % RH. decreased in 10 % steps down to 0 % RH, increased in 10 % steps up to 90 % RH and finally decreased in 10 % steps down to 0 % RH. In the experiments displayed in figure 8 and figure 9 the measurement cycle was started at 0 % RH, increased in 10 % steps up to 90 % RH and decreased in 10 % steps down to 0 % RH. The equilibrium condition for each step was set to a mass constancy of ± 0.01 % over 30 min. The temperature was 25± 0.1 °C.

| HPLC: | |
|---|---|
| Equipment: | liquid Chromatograph Waters 2695/Hewlett- Packard 1100 or similar; |
| Detection: | by UV at 210nm: |
| Column: | Waters XTerra MS C₁₈. 250 x 4.6 mm. 5 µm or similar; |
| Mobile phase: | |
| Mobile phase A: | Dissolve 1.80 grams of anhydrous sodium phosphate dibasic in 1000.0 ml of water. Adjust to pH 8.0 ±0.1 with phosphoric acid or NaOH 1N. |
| Mobile phase B: | Mix 750 ml of acetonitrile and 250 ml of methanol. |
| Temperature: | 60°C; |
| Injection Volume: | 50 µl; |
| Flow; | 0.9 ml/min: |
| Run Time: | 90 min; |
| Mode; | Gradient mode: |
| Gradient: | |

| Time [min] | %A | %B |
|---|---|---|
| 0 | 50 | 50 |
| 25 | 45 | 55 |
| 30 | 40 | 60 |
| 80 | 25 | 75 |
| 81 | 50 | 50 |
| 90 | 50 | 50 |

**Example 1**: Preparation of crystalline anhydrous form III of Tulathromycin A mixture of 1.33 g Tulathromycin and 3.64 ml isobutlymethyl ketone was stirred at 0 °C for 19 hours. The solid was collected by fixation and dried at 40 °C under vacuum for 7 hours. The thus obtained material was investigated by XRPD and found to be a novel crystalline form of Tulathromycin denominated as anhydrous form III.

**Example 2**: Preparation of crystalline anhydrous form III of Tulathromycin A mixture of 2 9 Tulathromycin in 6.1 ml isobutylmethyl ketone was stirred for 22 hours at 0 °C. Thereafter the solid was collected by filtration and dried under vacuum at 40 °C for 22 hours. The material was identified as anhydrous form III by XRPD. HPLC showed a Tulathromycin A content of 99.53 area% and a Tulathromycin B content of 0.20 area%.

**Example 3**: Preparation of crystalline form 11 of Tulathromycin 0.2 g of Tulathromycin form III obtained according to Example 1 were stored at about 80 % RH for 64 hours followed by storing the material at about 20 % RH for 4 hours. The thus obtained material was investigated by XRPD and found to be a novel crystalline form of Tulathromycin denominated as anhydrous form II.

**Reference Example 1**: Preparation of Tulathromycin monohydrate of WO 00/69874 1 g Tulathromycin was dissolved in 2.5 ml isobutylmethyl ketone and 50 µl water. To the clear solution seed crystals of Tulathromycin monohydrate (e.g. obtained by the method described in example 2 of WO 00/69874) were added and the obtained suspension was stirred at room temperature for about 20 hours. The solid was collected by filtration and dried under vacuum at 40 °C, The thus obtained material was investigated by XRPD and DSC and identified as Tulathromycin monohydrate of WO 00169874. A moisture sorotion/desorption curve of the material is displayed in figure 9.

## Claims

1. Crystalline anhydrous form II of Tulathromycin **characterized by** a XRPD pattern with peaks at 2-theta angles of 5.9± 0,2°, 6,7± 0,2°, 8.2 ± 0,2°,8.8± 0.2" and 9.5± 0.2°.

2. Crystalline anhydrous form II of Tulathromycin of claim 1, **characterized by** an IR spectrum with peaks at wavenumbers of 2934 ± 2 cm⁻², 1734± 2 cm⁻¹ 1456 ± 2 cm⁻¹, 1376± 2 cm⁻¹, 1331 ± 2 cm⁻¹. 1270± 2 cm⁻¹ 1174± 2 cm⁻¹, 1106± 2 cm⁻¹, 1053± 2 cm⁻¹ 996 ± 2 cm⁻¹, 972± 2 cm⁻¹ 898± 2 cm⁻¹, 858± 2 cm⁻¹, 834 ± 2 cm⁻¹ 805± 2 cm⁻¹, 697 ± 2 cm⁻¹ and 647± 2 cm⁻¹.

3. Crystalline anhydrous formII of Tulathromycin of claim 1 or 2, comprising a water content of 0-1.7 % ± 0.1 % between 0-90 % ± 1 % RH.

4. A process for the preparation of crystalline anhydrous form II of Tulathromycin of any of claims 1 to 3, comprising exposing crystalline anhydrous form III of Tulathromycin to a relative humidity of ≥ 40 %.

5. A process for the preparation of crystalline anhydrous form II of Tulathromycin of any one of claims 1 to 3, comprising the steps of:
a) mixing Tulathromycin in a dry solvent at ≤ 10°C;
b) isolating the solid material;
c) drying the isolated material to obtain crystalline form III of Tulathromycin;
d) subjecting the material to an atmosphere having 40-100 % RH; and
e) optionally removing water by subjecting the material to an atmosphere having 0-40 % RH;

6. Crystalline anhydrous form III of Tutathromycin **characterized by** an XRPD pattern with peaks at 2-theta angles of 6.4 ± 0,2°, 8.1 ± 0,2°, 8.4 ± 0.2°, 8.9 ± 0.2° and 9.3± 0.2°,

7. A process for the preparation of crystalline anhydrous form III of Tulathromycin of claim 6, comprising the steps of:
a) mixing Tulathromycin in a dry solvent at ≤ 10 °C;
b) isolating the solid material;
c) drying the isolated material to obtain crystalline form III of Tulathromycin;

8. A process according to claim 5 or 7, wherein the dry solvent comprises isobutylmethyl ketone.

9. Use of Tulathromycin form III in the manufacture of Tulathromycin form II.

10. Use of crystalline anhydrous form II of Tulathromycin of any one of claims 1 to 3 as an intermediate for the formulation of an anti-infective medicament

11. A pharmaceutical composition comprising crystalline anhydrous form II of Tulathromycin according to any one of claims 1-8 and a suitable carrier.

12. A pharmaceutical composition according to claim 11, further comprising a crystalline or amorphous pharmaceutical acceptable acid, in particular citric acid.

13. Use of crystalline anhydrous form III of Tulathromycin of claim 6 as an intermediate for the formulation of an anti-infective medicament.

14. A pharmaceutical composition comprising crystalline anhydrous form III of Tulathromycin of claim 6 and a suitable carrier.

15. A pharmaceutical composition according to claim 14, further comprising a crystalline or amorphous pharmaceutically acceptable acid, in particular citric acid.

16. A pharmaceutical composition comprising crystalline Tulathromycin and a solid crystalline or amorphous pharmaceutical acceptable acid.
